# EUROPEAN PATENT SPECIFICATION

(11) **EP 3 974 518 B1**
(45) Date of publication and mention of the grant of the patent: **06.08.2025**
(21) Application number: 20808832.8
(22) Date of filing: 19.05.2020
(51) Int. Cl.: C12N 5/07, G01N 33/68, G01N 33/574, C07K 1/14

(54) **METHOD FOR WASHING EXTRACELLULAR VESICLE**
VERFAHREN ZUM WASCHEN VON EXTRAZELLULÄREM VESIKEL
PROCÉDÉ DE LAVAGE DE VÉSICULE EXTRACELLULAIRE

(30) Priority: 21.05.2019 JP 2019095213
(43) Date of publication of application: 30.03.2022
(73) Proprietor: H.U. Group Research Institute G.K., Akiruno-shi, Tokyo 197-0833 (JP); Fujirebio Inc., Tokyo 107-0052 (JP)
(72) Inventor: GU, Ran, Tokyo 163-0410 (JP); ASAI, Fumi, Hachioji-shi, Tokyo 192-0031 (JP); INUZUKA, Tatsutoshi, Hachioji-shi, Tokyo 192-0031 (JP)
(74) Representative: Müller-Boré & Partner Patentanwälte PartG mbB
(86) International application number: PCT/JP2020/019808
(87) International publication number: WO 2020/235566

(56) References cited:
- WO-A1-2015/068772
- WO-A1-2018/062263
- WO-A1-2018/207932
- US-A1- 2019 117 792

## Description

### TECHNICAL FIELD

The present invention relates to a method of washing extracellular vesicle(s), and the like.

### BACKGROUND ART

An extracellular vesicle (EV) is a microscopic vesicle secreted from various types of cells and having a membrane structure, and exists in body fluids such as blood or cell culturing medium. The extracellular vesicles secreted extracellularly include exosomes, ectosomes, and apoptotic blebs. Since the extracellular vesicle refers to various groups that contain various substances that play a function such as intercellular signaling, it has been analyzed for the purposes of diagnosis, drug discovery and the like. Thus, it is required to develop a method of washing the extracellular vesicles useful for such analyses. For example, Patent Literature 1 describes a method of separating EV, in which a nonionic surfactant is added during immunoprecipitation and/or washing to reduce aggregation of solid-phase carriers.

### PRIOR ART REFERENCES

### Patent Literature

Patent Literature 1: International Patent Application Publication No. 2015/068772

### SUMMARY OF INVENTION

### PROBLEM TO BE SOLVED BY THE INVENTION

In the case of detecting a substances contained in an EV(s) in a specimen such as a body fluid, a large amount of the substances may be present outside the EV in the specimen. In general, the amount of target substances contained in the EV is quite smaller than the amount of the same substances present outside the EV in the specimen. Therefore, when the target substances in the EV recovered from the specimen is detected or measured, it is difficult to detect or measure the target substances in the EV because a large amount of the relevant substances existing outside the EV in the body fluid is contained as impurities to interfere background. For example, it is reported that diagnosis can be improved by measuring CEA that is a cancer marker in the EV. In the case of detecting or measuring CEA in the EV derived from the blood specimen, significantly larger amount of CEA is contained in the blood specimen than in EV, making it impossible to accurately detect or measure for CEA inside the EV that is the target substances when CEA existing outside the EV is adsorbed on the EV surface or absorbs to the container surface as an impurity.

Therefore, it is the object of the present invention to develop a method of reducing contamination with impurities in a system of operating extracellular vesicle(s).

### SOLUTION TO PROBLEM

As a result of an extensive study, the present inventors have found that a contamination amount of impurities (e.g., same substances as a target substances to be detected or measured) such as the same substances contained outside EV can be reduced by washing EV with a washing solution containing a predetermined nonionic surfactant, and the like, and completed the present invention.

In a first aspect the present invention relates to a method of washing an extracellular vesicle, the method comprising washing the extracellular vesicle with a nonionic surfactant, wherein the nonionic surfactant is an alcohol ethoxylate, and wherein the alcohol ethoxylate is a compound represented by formula (I): 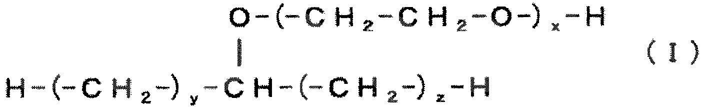
wherein x is 15 to 100;
y is an integer of 0 or more;
z is an integer of 0 or more;
y ≥ z; and
y + z is 5 to 30.

Typically the alcohol ethoxylate has an HLB of 15 or more, or 16 or less carbon atoms in a carbon chain. In some embodiments the nonionic surfactant is a polyoxyethylene-polyoxyalkylene block copolymer.

In some embodiments the polyoxyethylene-polyoxyalkylene block copolymer is a polyoxyethylene-polyoxypropylene block copolymer represented by formula (III):

HO-(-CH₂-CH₂-O-)ₓ₂-((-CH(-CH₃))CH₂-O-)_{y2}- (-CH₂-CH₂-O-)_{z2}-H (III)

wherein x2 is an integer of 1 or more;
y2 is 10 to 100;
z2 is an integer of 1 or more; and
x2 + z2 is 20 to 300.

Typically the extracellular vesicle is treated with 0.001 to 10.0 w/v% of the nonionic surfactant.

In general, the extracellular vesicle is an exosome.

Washing the extracellular vesicle with the nonionic surfactant is washing a complex of the extracellular vesicle and an extracellular vesicle membrane-binding substance with the nonionic surfactant.

Typically the extracellular vesicle membrane-binding substance is an antibody against a tetraspanin membrane protein.

In a second aspect the present invention relates to a method of producing a purified extracellular vesicle, the method comprising:
(1) washing an extracellular vesicle with a nonionic surfactant as described in the first aspect of the present invention; and
(2) separating the washed extracellular vesicle.

Additionally the method can comprise the following steps:
(1') treating the extracellular vesicle with an extracellular vesicle membrane-binding substance to form a complex of the extracellular vesicle and the extracellular vesicle membrane-binding substance;
(2') washing the complex with the nonionic surfactant; and
(3') separating the complex.

In a third aspect the present invention relates to a method of analyzing an extracellular vesicle, the method comprising analyzing the extracellular vesicle washed or purified by the methods described herein.

Typically the method of analyzing an extracellular marker additionally involves the detection of an extracellular vesicle inside marker, wherein the extracellular vesicle inside marker can be a carcinoembryonic antigen (CEA) or CA125.

Specifically the method of analyzing an extracellular marker can include the following steps:
(1) disrupting the washed or purified extracellular vesicle; and
(2) detecting an extracellular vesicle inside marker of the disrupted extracellular vesicle.

In a fourth aspect the present invention relates to a kit comprising:
(1) a nonionic surfactant having a structure as defined in the first aspect of the present invention; and
(2) an extracellular vesicle membrane-binding substance.

### EFFECT OF THE INVENTION

According to the present invention, it is possible to reduce contamination with impurities by washing the extracellular vesicle with the predetermined nonionic surfactant. The reduction of contamination with impurities enables reduction of problems in a background and the like during detection and measurement for an extracellular vesicle marker, improving accuracy of detection and measurement. Therefore, the present invention is effective in analysis or diagnosis at a high accuracy when the extracellular vesicle marker is used as an indicator. The present invention is effective also in recovering the extracellular vesicle serving as a sample for the analysis or diagnosis at a high accuracy.

### BRIEF DESCRIPTION OF DRAWINGS

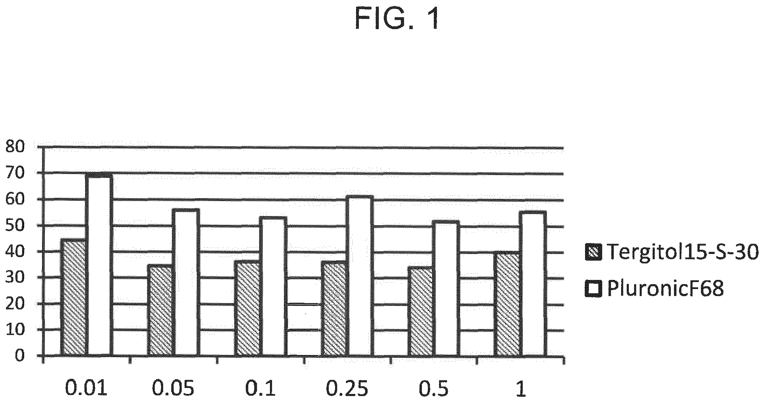

FIG. 1 is a graph representing comparison of concentration-dependent washing effect of Tergitol 15-s-30 and Pluronic F68 as indicators of residual total protein in recovering of an EV in Example 6.

### EMBODIMENTS FOR CARRYING OUT THE INVENTION

### 1. Classification of surfactant

In the present invention, plural kinds of surfactant can be used depending on one or more objects (e.g., washing EV and disrupting EV). As such, for the requirement of selection of surfactant suitable for plural objects, the surfactant is classified according to chemical property (ionic property at hydrophilic moieties) and functional property (property/use for EV).

### 1-1. Classification of surfactant based on ionic property at hydrophilic moieties

The surfactants can be classified into nonionic surfactants, cationic surfactants, anionic surfactants and zwitterionic surfactants based on ionic properties at hydrophilic moieties. Each surfactant is exemplified below.

### 1-1-1. Nonionic surfactant

Examples of the nonionic surfactant include polyoxyethylene alcohol structure-containing nonionic surfactants (e.g., alcohol ethoxylates, polyoxyethylene-polyoxyalkylene block copolymers), polyoxyethylene sorbitan fatty acid esters (for example, TWEEN (registered trademark) series (e.g., TWEEN 20, TWEEN 40, TWEEN 80)), polyoxyethylene octylphenyl ether (for example, TRITON (registered trademark) series (e.g., Triton X-100, Triton X-114, Triton X-305, Triton X-405 and Triton X-705)), and N-D-gluco-N-methylalkanamide (for example, MEGA series (e.g., MEGA 8 and MEGA 10).

"Polyoxyethylene alcohol structure-containing nonionic surfactant" is a chain compound (compound not containing cyclic structure) containing a structure represented by -O- (-CH₂-CH₂-O-)ₓ-H, wherein X is an integer of 1 or more (hereinafter referred to as a "polyoxyethylene alcohol structure"). Such a compound may be either a linear compound or a branched chain compound. Such a compound may be formed by single bonds or may contain a multiple bond(s). Such a compound may contain one or more (e.g., 2, 3 and 4) polyoxyethylene alcohol structure(s). Examples of structures other than the polyoxyethylene alcohol structure in such compounds include alkylene structures (linear or branched chain), and polyoxyalkylene structures (linear or branched chain). Such compounds may be composed of a carbon atom(s), a hydrogen atom(s), and an oxygen atom(s). Examples of such compounds include alcohol ethoxylates and polyoxyethylene-polyoxyalkylene block copolymers (e.g., polyoxyethylene-polyoxypropylene block copolymer).

The term "alcohol ethoxylate" refers to a group of compounds represented by the following formula (I):

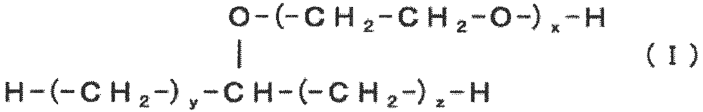

In the formula, x is an integer of 1 or more;
y is an integer of 0 or more;
z is an integer of 0 or more; and
y ≥ z.

In formula (I), a moiety represented by "H-(-CH₂-)_{y}-CH-(-CH₂-)_{z}-H" may be referred to as "hydrophobic side chain", "carbon chain", or "alkyl chain". In formula (I), the number of the carbon atoms in the hydrophobic side chain corresponds to y + z + 1. In formula (I), a moiety represented by "-(-CH₂-CH₂-O-)ₓ-H" may be referred to as "hydrophilic side chain" or "ethoxylate chain".

Examples of alcohol ethoxylates include branched chain alcohol ethoxylates and linear alcohol ethoxylates.

The branched chain alcohol ethoxylate corresponds to a compound represented by the above formula (I) in which y is an integer of 1 or more and z is an integer of 1 or more. Examples of the branched-chain alcohol ethoxylate include polyoxyethylene (3) sec-tridecylether, polyoxyethylene (7) sec-tridecylether, polyoxyethylene (9) sec-tridecylether, polyoxyethylene (12) sec-tridecylether, polyoxyethylene (15) sec-tridecylether, polyoxyethylene (20) sec-tridecylether, polyoxyethylene (30) sec-tridecylether, and polyoxyethylene (40) sec-tridecylether. Examples of the branched chain alcohol ethoxylate include TERGITOL (registered trademark) series such as Tergitol 15-S-3, Tergitol 15-S-5, Tergitol 15-S-7, Tergitol 15-S-9, Tergitol 15-S-12, Tergitol 15-S-15, Tergitol 15-S-20, Tergitol 15-S-30 and Tergitol 15-S-40 as commercial products. In the TERGITOL 15-S series, the number of carbon atoms in an alkyl chain (y + z + 1) is 11 to 15 (13 in average), and the number subsequent to "S" in the system name represents the unit number (x) of ethylene oxide in the ethoxylate chain.

The linear alcohol ethoxylate corresponds to a compound represented by the above formula (I) in which z is 0. Examples of the linear alcohol ethoxylate include polyoxyethylene (4) laurylether, polyoxyethylene (23) laurylether, polyoxyethylene (20) cetylether, polyoxyethylene (20) stearylether. Examples of commercial products of the linear alcohol ethoxylate include BRIJ (registered trademark) series compounds such as Brij30, Brij35, Brij58, and Brij78. The linear alcohol ethoxylate can be represented also by the following formula (II):

H-(-CH₂-)ₙ-O-(-CH₂-CH₂-O-)ₓ-H (II)

In the formula, x is an integer of 1 or more; and
n is an integer of 1 or more.

The term "polyoxyethylene-polyoxyalkylene block copolymer" refers to a block copolymer containing a polyoxyethylene block and a polyoxyalkylene block. Examples of the polyoxyethylene-polyoxyalkylene block copolymer include a polyoxyethylene-polyoxypropylene block copolymer.

The "polyoxyethylene-polyoxypropylene block copolymer" refers to a group of compounds represented by the following formula (III):

HO-(-CH₂-CH₂-O-)ₓ₂-((-CH(-CH₃))CH₂-O-)_{y2}- (-CH₂-CH₂-O-)_{z2}-H (III)

In the formula, each of x2, y2 and z2 is an integer of 1 or more.

Examples of the polyoxyethylene-polyoxypropylene block copolymer include poloxamer 182, poloxamer 108, poloxamer 188, poloxamer 217, poloxamer 237, poloxamer 238, poloxamer 288, poloxamer 388, and poloxamer 407. Examples of commercial products of the polyoxyethylene-polyoxypropylene block copolymer include PLURONIC (registered trademark) series compounds such as Pluronic L62, Pluronic F38, Pluronic F68, Pluronic F77, Pluronic F87, Pluronic F88, Pluronic F98, Pluronic F108 and Pluronic F127.

### 1-1-2. Cationic surfactant

Examples of the cationic surfactant include quaternary ammonium salts. Examples of the salts include salts of halogen (e.g., fluorine, chlorine, bromine and iodine). Examples of the quaternary ammonium salt include Cn alkyltrimethylammonium bromide (CnTAB), and Cn alkyltrimethylammonium chloride (CnTAC). Examples of the CnTAB include octyltrimethylammonium bromide (C8TAB), nonyltrimethylammonium bromide (C9TAB), dodecyltrimethylammonium bromide (C12TAB), tetradecyltrimethylammonium bromide (C14TAB), and hexadecyltrimethylammonium bromide (C16TAB). Examples of the CnTAC include dodecyltrimethylammonium chloride (C12TAC), tetradecyltrimethylammonium chloride (C14TAC), hexadecyltrimethylammonium chloride (C16TAC), and octadecyltrimethylammonium chloride (C18TAC).

### 1-1-3. Anionic surfactant

Examples of the anionic surfactant include carboxylic acid type surfactants (e.g., N-decanoylsarcosine sodium (NDS), and N-lauroylsarcosine sodium hydrate (NLS)), sulfonic acid type surfactants (e.g., sodium 1-nonansulfonate (NSS), and sodium dodecylbenzenesulfonate (SDBS)), carboxylic acid type-sulfonic acid type surfactants (e.g., sodium chondroitin sulfate (CSSS)), and sulfate ester type surfactants (e.g., sodium dodecyl sulfate (SDS)).

### 1-1-4. Zwitterionic surfactant

Examples of the zwitterionic surfactant include quaternary ammonium-sulfonic acid type surfactants. Examples of the quaternary ammonium-sulfonic acid type surfactants include 3-[(3-cholamidopropyl) dimethylammonio]-1-propane sulfonate (CHAPS), 3-[(3-cholamidopropyl) dimethylammonio]-2-hydroxy-1-propane sulfonate (CHAPSO), 3-(N,N-dimethyloctylammonio) propane sulfonate (C8APS), 3-(decyldimethylammonio) propane sulfonate (C10APS), N-dodecyl-N,N-dimethyl-3-ammonio-1-propane sulfonate (C12APS), 3-(N,N-dimethylmyristylammonio) propane sulfonate (C14APS), 3-(N,N-dimethylpalmitylammonio) propane sulfonate (C16APS), dimethylethylammonium propane sulfonate (NDSB-195), 3-[dimethyl-(2-hydroxyethyl) ammonio]-1-propane sulfonate (NDSB-211), and 3-(benzenedimethylammonio) propane sulfonate (NDSB-256).

### 1-1-5. Physical property value of surfactant

HLB (Hydrophilic-Lipophilic Balance) value can be used as a physical property value representing hydrophilicity and lipophilic properties of the surfactant. The HLB value closer to zero indicates higher lipophilicity. The HLB value of the commercial compound may be the value described in the manufacturer's data sheet. The HLB value described in the manufacturer's data sheet may exceed 20. The HLB value of the alcohol ethoxylate may be determined simply by a griffin method, for example.

### 1-1-6. Summary of classification of surfactant based on ionic property

The classification of the surfactants based on ionic properties at the hydrophilic moieties is summarized as follows.

**Table 1. The classification of the surfactants based on ionic properties at the hydrophilic moieties**

| Classification | Name | Hydrophobic side chain* | HLB | CAS No. |
|---|---|---|---|---|
| Nonionic surfactants | TWEEN20 | 11 | 16.7 | 9005-64-5 |
| | TWEEN40 | 15 | 15.6 | 9005-66-7 |
| | TWEEN80 | 17 | 15 | 9005-65-6 |
| | Triton X-100 | Octylphenyl ether | 13.5 | 9002-93-1 |
| | Triton X-114 | Octylphenyl ether | 12.4 | 9036-19-5 |
| | Triton X-305 | Octylphenyl ether | 17.3 | 9002-93-1 |
| | Triton X-405 | Octylphenyl ether | 17.6 | 9036-19-5 |
| | Triton X-705 | Octylphenyl ether | 18.4 | 9036-19-5 |
| | Tergitol 15-S-3 | 13 | 8.0 | 84133-50-6 |
| | Tergitol 15-S-5 | 13 | 10.5 | 84133-50-6 |
| | Tergitol 15-S-7 | 13 | 12.1 | 84133-50-6 |
| | Tergitol 15-S-9 | 13 | 13.3 | 84133-50-6 |
| | Tergitol 15-S-12 | 13 | 14.5 | 84133-50-6 |
| | Tergitol 15-S-15 | 13 | 15.4 | 68131-40-8 |
| | Tergitol 15-S-20 | 13 | 16.3 | 68131-40-8 |
| | Tergitol 15-S-30 | 13 | 17.4 | 68131-40-8 |
| | Tergitol 15-S-40 | 13 | 18 | 68131-40-8 |
| | MEGA 8 | 7 | 8.6 | 85316-98-9 |
| | MEGA 10 | 9 | 8.4 | 85261-20-7 |
| | Brij 58 | 16 | 15.7 | 9004-95-9 |
| | Brij 35 | 12 | 16.9 | 9002-92-0 |
| | Brij 30 | 12 | 9.7 | 9002-92-0 |
| | Brij 78 | 18 | 15.3 | 9005-00-9 |
| | Pluronic L62 | (Block copolymer) | 1-7** | 9003-11-6 |
| | Pluronic F68 | (Block copolymer) | >24** | 9003-11-6 |
| | Pluronic F87 | (Block copolymer) | >24** | 9003-11-6 |
| | Pluronic F88 | (Block copolymer) | >24** | 9003-11-6 |
| | Pluronic F98 | (Block copolymer) | >24** | 9003-11-6 |
| | Pluronic F108 | (Block copolymer) | >24** | 9003-11-6 |
| | Pluronic F127 | (Block copolymer) | 18-23** | 9003-11-6 |
| Cationic surfactants | C8TAB | 8 | | 2083-68-3 |
| | C9TAB | 9 | | 1943-11-9 |
| | C12TAB | 12 | | 1119-94-4 |
| | C14TAB | 14 | | 1119-97-7 |
| | C16TAB | 16 | | 57-09-0 |
| | C12TAC | 12 | | 112-00-5 |
| | C14TAC | 14 | | 4574-04-3 |
| | C16TAC | 16 | | 112-02-7 |
| | C18TAC | 18 | | 112-03-8 |
| Anionic surfactants | NDS | 9 | | 30377-07-2 |
| | NLS | 11 | | 137-16-6 |
| | NSS | 9 | | 35192-74-6 |
| | CSSS | | | 9082-07-9 |
| | SDBS | 12 | | 25155-30-0 |
| Zwitterionic surfactant | CHAPS | | | 75621-03-3 |
| | C8APS | 8 | | 15178-76-4 |
| | C10APS | 10 | | 15163-36-7 |
| | C12APS | 12 | | 14933-08-5 |
| | C14APS | 14 | | 14933-09-6 |
| | C16APS | 16 | | 2281-11-0 |
| | NDSB-195 | 2 | | 160255-06-1 |
| | NDSB-211 | Propanesulfonic acid | | 38880-58-9 |
| | NDSB-256 | Benzyldimethyl | | 81239-45-4 |

| | | | | |
|---|---|---|---|---|
| *Number indicates the number of the carbon atoms when hydrophobic side chain is alkyl chain. **The value described in the manufacturer's data sheet. The vale exceed 20. | | | | |

### 1-2. Classification of surfactant based on characteristic and use for EV

The surfactants can be classified into "EV-nondisruptive surfactants" and "EV-disruptive surfactants" depending on the extent of disrupting the EV. The "EV-nondisruptive surfactants" can be further classified into "EV-washable surfactants" for surfactants with washing effects. With selectin of the "EV-washable surfactants" among the "EV-nondisruptive surfactants", it is possible to wash the EV and an EV operation system while preventing an EV inside marker from being discharged outside the EV due to the destruction of the EV. Nonionic surfactants among the "EV-washable surfactants" are referred to as "EV-washable nonionic surfactants". The above classifications can be performed according to the methods described in Examples of this description.

### 2. Method for washing extracellular vesicle

The present invention provides a method of washing an extracellular vesicle(s).

The extracellular vesicle is a microscopic vesicle secreted from various types of cells and having a membrane structure. Examples of the extracellular vesicle include exosomes, ectosomes and apoptotic blebs. Preferably, the extracellular vesicle is the exosome. The extracellular vesicle can also be defined by its size. The size of the extracellular vesicle is, for example, 30 to 1000 nm, preferably 50 to 300 nm, and more preferably 80 to 200 nm. The size of the extracellular vesicle can be measured by, for example, a method based on Brownian movement of the extracellular vesicle, a light scattering method, and an electric resistance method, and the like. Preferably, the size of the extracellular vesicle is measured by NanoSight (manufactured by Malvern Instruments).

The method of washing an extracellular vesicle(s) of the present invention includes washing the extracellular vesicle(s) with the "EV-washable nonionic surfactant". The "EV-washable nonionic surfactant" is the polyoxyethylene alcohol structure-containing nonionic surfactant.

The polyoxyethylene alcohol structure-containing nonionic surfactant used as the "EV-washable nonionic surfactant" in the washing method of the present invention is a chain compound (compound not having cyclic structure) that contains a structure (polyoxyethylene alcohol structure) represented by -O-(-CH₂-CH₂-O-)ₓ-H. x is an integer of 1 or more and may be 1 to 300, for example. Such a compound may be either a linear compound or a branched chain compound. Such a compound may be formed by single bonds or may contain a multiple bond(s), and is preferably formed by single bonds. Such a compound may contain one or plural (e.g., 2, 3 or 4) polyoxyethylene alcohol structure(s). Examples of structures other than the polyoxyethylene alcohol structure in such a compound include alkyl structures (linear or branched chain), and polyoxyalkylene structures (linear or branched chain). The alkyl structure may be a C₆₋₃₁ alkyl structure, for example, and is preferably a C₆₋₃₁ linear alkyl structure. The alkylene in the polyoxyalkylene structure may be a C₁₋₆ alkylene group, for example. Examples of the C₁₋₆ alkylene group include a C₁ alkylene group (methylene group), a C₂ alkylene group (ethylene group and ethylidene group), a C₃ alkylene group (propylidene group, propylene group, and trimethylene group, isopropylidene group), a C₄ alkylene group (e.g., tetramethylene group), a C₅ alkylene group (e.g., a pentamethylene group), a C₆ alkylene group (e.g., hexamethylene group). Such a compound may be formed of a carbon atom(s), a hydrogen atom(s), and an oxygen atom(s). Examples of such a compound include alcohol ethoxylates and polyoxyethylene-polyoxyalkylene block copolymers (e.g., polyoxyethylene-polyoxypropylene block copolymers).

The "alcohol ethoxylate" is a compound represented by the following formula (I):

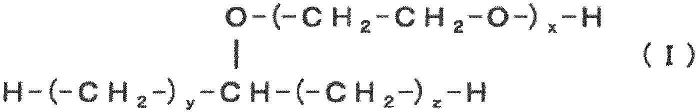

In the formula, x is an integer of 1 or more;
y is an integer of 0 or more;
z is an integer of 0 or more; and
y ≥ z.

The alcohol ethoxylate used as the EV-washable nonionic surfactant in the washing method of the present invention may be either a branched chain alcohol ethoxylate or a linear alcohol ethoxylate.

Such a branched chain alcohol ethoxylate corresponds to a compound represented by the above formula (I) in which y is an integer of 1 or more and z is an integer of 1 or more. Examples of such a branched chain alcohol ethoxylate include polyoxyethylene (7) sec-tridecylether, polyoxyethylene (9) sec-tridecylether, polyoxyethylene (15) sec-tridecylether, polyoxyethylene (20) sec-tridecylether, polyoxyethylene (30) sec-tridecyl ether, and polyoxyethylene (40) sec-tridecylether. Examples of such a branched-chain alcohol ethoxylate include TERGITOL (registered trademark) series compounds such as Tergitol 15-S-7, Tergitol 15-S-9, Tergitol 15-S-15, Tergitol 15-S-20, Tergitol 15-S-30 and Tergitol 15-S-40 as commercial products.

Such a linear alcohol ethoxylate corresponds to a compound represented by the above formula (I) in which z is 0. Examples of such a linear alcohol ethoxylate include BRIJ (registered trademark) series compounds such as Brij35, Brij58, and Brij78, as commercial products. The linear alcohol ethoxylate can be represented also by the following formula (II).

H-(-CH₂-)ₙ-O-(-CH₂-CH₂-O-)ₓ-H (II)

In the formula, x is an integer of 1 or more; and
n is an integer of 1 or more.

The alcohol ethoxylate used as the EV-washable non-ionic surfactant in the washing method of the present invention is preferably a compound represented by formula (I) in which
x is 15 to 100;
y is an integer of 0 or more;
z is an integer of 0 or more;
y ≥ z; and
y + z is 5 to 30.

The alcohol ethoxylate used as the EV-washable nonionic surfactant in the washing method of the present invention is preferably an alcohol ethoxylate that has an HLB of 15 or more, or 16 or less carbon atoms in a carbon chain. The alcohol ethoxylate used in the washing method of the present invention is still more preferably an alcohol ethoxylate having an HLB of 15 or more. Preferred examples of the alcohol ethoxylate used in the washing method of the present invention include Tergitol 15-S-15, Tergitol 15-S-20, Tergitol 15-S-30, Tergitol 15-S-40, Brij35, Brij58 and Brij78. More preferred examples of the alcohol ethoxylate used in the washing method of the present invention include Tergitol 15-S-30, Tergitol 15-S-40, Brij35, and Brij58.

The "polyoxyethylene-polyoxyalkylene block copolymer" is a block copolymer that contains a polyoxyethylene block or polyoxyethylene blocks and a polyoxyalkylene block or polyoxyalkylene blocks. The alkylene in the polyoxyalkylene block may be a C₁₋₆ alkylene group, for example. Examples of the C₁₋₆ alkylene group include a C₁ alkylene group (methylene group), a C₂ alkylene group (ethylene group and ethylidene group), a C₃ alkylene group (propylidene group, propylene group, trimethylene group and isopropylidene group), a C₄ alkylene group (e.g., tetramethylene group), a C₅ alkylene group (e.g., pentamethylene group), and a C₆ alkylene group (e.g., hexamethylene group). Examples of the polyoxyethylene-polyoxyalkylene block copolymer include a block copolymer having a structure of HO-[polyoxyethylene block]-[polyoxyalkylene block]-[polyoxyethylene block]-H. The polyoxyethylene-polyoxyalkylene block copolymer may be preferably a polyoxyethylene-polyoxypropylene block copolymer.

The "polyoxyethylene-polyoxypropylene block copolymer" is a group of compounds represented by the following formula (III):

HO-(-CH₂-CH₂-O-)ₓ₂-((-CH(-CH₃))CH₂-O-)_{y2}- (-CH₂-CH₂-O-)_{z2}-H (III)

In the formula, each of x2, y2 and z2 is an integer of one or more.

Examples of the polyoxyethylene-polyoxypropylene block copolymer include poloxamer 188 and poloxamer 407. Examples of commercial products of the polyoxyethylene-polyoxypropylene block copolymer include PLURONIC (registered trademark) series compounds such as Pluronic F68 and Pluronic F127.

The polyoxyethylene-polyoxypropylene block copolymer used as the EV-washable non-ionic surfactant in the washing method of the present invention is preferably a compound represented by formula (III) in which
x2 is an integer of 1 or more;
y2 is 10 to 100;
z2 is an integer of 1 or more; and
x2 + z2 is 20 to 300.

The polyoxyethylene-polyoxypropylene block copolymer used as the EV-washable nonionic surfactant in the washing method of the present invention is more preferably a polyoxyethylene-polyoxypropylene block copolymer having an HLB of 15 or more. Preferred examples of the polyoxyethylene-polyoxypropylene block copolymer used in the washing method of the present invention include poloxamer 188 (e.g., Pluronic F68), poloxamer 108 (e.g., Pluronic F38), poloxamer 217 (e.g., Pluronic F77), poloxamer 237 (e.g., Pluronic F87), poloxamer 238 (e.g., Pluronic F88), poloxamer 288 (e.g., Pluronic F98), poloxamer 388 (e.g., Pluronic F108), and poloxamer 407 (e.g., Pluronic F127). More preferred examples of the polyoxyethylene-polyoxypropylene block copolymer used in the washing method of the present invention include poloxamer 188 (e.g., Pluronic F68) and poloxamer 407 (e.g., Pluronic F127).

The nonionic surfactant used as the EV-washable nonionic surfactant in the washing method of the present invention is preferably alcohol ethoxylate.

The concentration of the EV-washable nonionic surfactant in the washing of the extracellular vesicle(s) is not particularly limited as long as it is possible to inhibit the adsorption of contaminants on the extracellular vesicle(s) and the system for operating the extracellular vesicle(s) and to inhibit the destruction of extracellular vesicle(s). Such a concentration varies depending on the type of the EV-washable nonionic surfactant, and may be 0.001 to 10.0 w/v%, for example. Preferably, the concentration of the EV-washable nonionic surfactant may be 0.002 w/v% or more, 0.005 w/v% or more, or 0.01 w/v% or more. Such a concentration varies depending on the type of EV-washable nonionic surfactant, and may be 7.5 w/v% or less, 5.0 w/v% or less, or 2.0 w/v% or less. More preferably, the concentration of the EV-washable nonionic surfactant may be 0.002 to 7.5 w/v%, 0.005 to 5.0 w/v%, or 0.01 to 2.0 w/v%.

The EV-washable nonionic surfactant used in the washing method of the present invention may be an aqueous solution. Examples of the aqueous solution include water (e.g., distilled water, sterilized water, sterilized distilled water and pure water) and buffer. The buffer is preferred. Examples of the buffer include phosphate buffer, phosphate-buffered saline (PBS), tartrate buffer, citrate buffer, acetate buffer, glycine buffer, carbonate buffer, 2-morpholinoethanesulfonic acid (MES) buffer, tris(hydroxymethyl)aminomethane (Tris) buffer, borate buffer, 3-morpholinopropanesulfonic acid (MOPS) buffer, N,N-bis(2-hydroxyethyl)glycine (Bicine) buffer, bis(2-hydroxyethyl)iminotris(hydroxymethyl) methane (Bis-Tris) buffer and 2-[4-(2-hydroxyethyl)1-piperazinylethanesulfonic acid (HEPES) buffer. It is preferable that the buffer has neutral pH. More specifically, such a pH is preferably 5.0 or more, more preferably 5.5 or more, and still more preferably 6.0 or more. Also, the pH is preferably 9.0 or less, more preferably 8.5 or less, and still more preferably 8.0 or less. The pH can be measured by well-known methods in the relevant field. Preferably, it is possible to employ a value measured at 25°C with a pH meter having a glass electrode as the pH.

The temperature for washing the extracellular vesicle(s) may be 4 to 45°C, for example, and preferably 15 to 40°C. The time for washing is not particularly limited as long as it is sufficient to inhibit the adsorption of impurities on the extracellular vesicle(s), and may be 1 second or more, 5 seconds or more, 10 seconds or more, or 20 seconds or more, for example. Also, such a time may be 4 minutes or less, 2 minutes or less, or 1 minute or less. In the washing of the extracellular vesicle(s), after the mixing the extracellular vesicle(s) with the EV-washable nonionic surfactant, the mixture may be left to stand or agitated by a vortex mixer or the like, or further mixed by pipetting operation.

In the present invention, the "washing of extracellular vesicle(s)" refers to reducing the amount of impurities in the extracellular vesicle(s) and the system (e.g., solution and container) for operating the extracellular vesicle(s). Examples of the impurity include proteins, nucleic acids (e.g., RNA and DNA), saccharides, lipids, amino acids, vitamins, polyamines, and peptides. The impurity refers to a contaminant that interferes with the analysis of the extracellular vesicle(s). Examples of the contaminant include impurities (e.g., substances that are identical or similar to the detection target) that enhance the detection background, and impurities that attenuate the detection signal (e.g., substances that inhibit the detection of the detection object). The washing of the extracellular vesicle(s) preferably reduces the amount of substance that is the same or similar to that of the detection object, and more preferably reduces the total amount of substance belonging to the same category as the detection object (e.g., the total protein amount when the detection object is a protein). Examples of the operation of the extracellular vesicle(s) include washing the extracellular vesicle(s), producing a purified extracellular vesicle(s) (i.e., recovery of the extracellular vesicle(s)), and the analysis of the extracellular vesicle(s). Examples of the container for operating the extracellular vesicle(s) include a container that is made of a substance such as plastic (e.g., polypropylene), glass and the like, and the container made of plastic is preferred.

The extracellular vesicle(s) washed by the washing method of the present invention can be used for analysis. The analysis of the extracellular vesicle(s) may be an analysis of a component (EV marker) contained in the extracellular vesicle(s), for example. Examples of the EV marker include an EV inside marker and an EV surface marker. Examples of the EV inside marker include carcinoembryonic antigen (CEA), CA125, CA15-3, actin family, TSG101, ALIX, Charged multivesicular body protein (CHMP) family, Glyceraldehyde 3-phosphate dehydrogenase (GAPDH), poly (ADP-ribose) polymerase (PARP) family, AFP, CA19-9, Flotillin-I, Flotillin-II, Rab protein, programmed cell death (PDCD) 6, phospholipid scramblase (PLSCR) family, cytochrome C oxidase (COX) family, lamin family, proliferating cell nuclear antigen (PCNA), tubulin family, TATA binding protein (TBP), voltage dependent anionic channel protein (VDAC), amyloid beta, and tau protein. Examples of the EV surface marker include a tetraspanin membrane protein (extracellular vesicle membrane specific four transmembrane proteins, e.g., CD9, CD63 and CD81), an extracellular matrix metalloproteinase inducer (CD147), heat shock protein (HSP) 70, HSP90, major histocompatibility complex (MHC) I, lysosome associated membrane protein (LAMP) 1, intercellular adhesion molecule (ICAM)-1, integrin, ceramide, cholesterol, phosphatidylserine, Annexins, Caveolin-I and EpCAM.

The washing method of the present invention may be carried out in combination with a precipitation method (e.g., immunoprecipitation method) using an extracellular vesicle membrane-binding substance . In this case, it is possible to form a complex (e.g., immunoprecipitated complex) of the extracellular vesicle(s) and the extracellular vesicle membrane-binding substance (e.g., antibody) by the precipitation method, and then wash the complex with the EV-washable nonionic surfactant. That is, the washing of the extracellular vesicle(s) with the EV-washable nonionic surfactant may refer to washing the complex of the extracellular vesicle(s) and the extracellular vesicle membrane-binding substance with the EV-washable nonionic surfactant.

The extracellular vesicle membrane-binding substance used in the present invention is a substance with an affinity to the EV surface marker described above. Examples of the extracellular vesicle membrane-binding substance include the antibodies (e.g., monoclonal antibodies and polyclonal antibodies) against the EV surface marker, and antigen-binding fragments thereof, aptamers, phosphatidylserine-bound proteins, and ceramidebound proteins. The antigen-binding fragment is antigen fragment that maintains capability of binding to the targeted EV surface marker, and may be Fab, Fab', F(ab')₂, scFv or the like. The extracellular vesicle membrane-binding substance is preferably the antibody or the antigen-binding fragment thereof, and more preferably the monoclonal antibody or the antigen-binding fragment thereof. The extracellular vesicle membrane-binding substance used in the present invention may be single substance or a combination of plural substances .

The extracellular vesicle membrane-binding substance may be bound to the solid phase facilitating the separation of the extracellular vesicles. As the solid phase, it is possible to use sepharose beads, agarose beads, magnetic beads (magnetic particles) or a plastic plate, for example. The extracellular vesicle membrane-binding substance can be solid-phased to the solid phase in a conventional method known to a person skilled in the art.

### 3. Method for producing purified extracellular vesicle

The present invention also provides a method for producing purified extracellular vesicle(s).

The production method of the present invention includes the following steps:
(1) washing the extracellular vesicle(s) with the "EV-washable nonionic surfactant"; and
(2) separating the washed extracellular vesicle(s).

The separation of the extracellular vesicle(s) in the step (2) can be carried out by the precipitation method using the extracellular vesicle membrane-binding substance, or ultracentrifugation, for example. The extracellular vesicle(s) can be recovered by precipitating the extracellular vesicle(s) by the precipitation method or the ultracentrifugation, and then collecting the precipitation or discarding the supernatant. The collection of the precipitation can be carried out by any method known in the relevant field (e.g., magnetically collecting magnetic particles, centrifugation of sepharose beads). The separation is preferably isolation or purification.

When the separation of the extracellular vesicle(s) in the step (2) is performed by the precipitation method, the complex of the extracellular vesicle(s) and the extracellular vesicle membrane-binding substance may be formed prior to the step (2). That is, the production method of the present invention in this case may be performed by the following steps:
(1') treating the extracellular vesicle(s) with the extracellular vesicle membrane-binding substance to form the complex of the extracellular vesicle(s) and the extracellular vesicle membrane-binding substance;
(2') washing the complex of the extracellular vesicle(s) and the extracellular vesicle membrane-binding substance with the "EV-washable nonionic surfactant"; and
(3') separating the complex of the extracellular vesicle(s) and the extracellular vesicle membrane-binding substance.

In the production method of the present invention, the purified extracellular vesicle(s) can be obtained as the complex of the extracellular vesicle(s) and the extracellular vesicle membrane-binding substance or free extracellular vesicle(s). For obtaining the purified extracellular vesicle(s) as the free form, the production method of the present invention may include a step of dissociating the extracellular vesicle(s) from the complex. The dissociation of the extracellular vesicle(s) from the complex can be carried out by any method known in the relevant field.

The kind of the "EV-washable nonionic surfactant" used in the production method of the present invention, each condition in the treatment with the "EV washable nonionic surfactant", and the extracellular vesicle membrane-binding substance are defined by the washing method of the present invention.

The production method of the present invention can be used as a method for recovering, isolating or purifying the extracellular vesicle(s).

With the reduced contamination amount of the impurities, the purified extracellular vesicle(s) produced by the production method of the present invention may be used as an analyzed object for analysis, or may be used as an EV standard product.

### 4. Method for analyzing extracellular vesicle

The present invention also provides a method for analyzing the extracellular vesicle(s).

The analysis method of the present invention includes analyzing the extracellular vesicle(s) washed by the washing method of the present invention or the purified extracellular vesicle(s) produced by the production method of the present invention (both referred to as "extracellular vesicles obtained in the present invention", hereinafter).

The analysis of the extracellular vesicle(s) may be an analysis of a component (e.g., the EV inside marker and the EV surface marker) contained in the extracellular vesicle(s), and may be an analysis of the particle of the extracellular vesicle itself. The analysis of the extracellular vesicle(s) is preferably the analysis of the component contained in the extracellular vesicle(s), and more preferably the analysis of the EV inside marker. With the reduced contamination amount of the impurities, the extracellular vesicle(s) obtained in the present invention can be suitably used for the analysis of the component (e.g., EV inside marker) contained in the extracellular vesicle(s).

For the analysis of the components contained in the extracellular vesicle, the analysis refers to detection or quantification of the components. Also, such an analysis refers to an analysis of one component or plural components. Examples of the components to be analyzed include proteins, nucleic acids (e.g., RNA and DNA), saccharides, lipids, amino acids, vitamins, polyamines and peptides.

The component analysis can be performed by any method known in the relevant field.

In the case that the component to be analyzed is a protein, examples of the analysis method include immunoassay and mass spectrometry. Examples of the immunoassay include a direct competitive method, an indirect competitive method and a sandwich method. Also, examples of such an immunoassay include chemiluminescent immunoassay (CLIA) [e.g., a chemiluminescent enzyme immunoassay (CLEIA)], turbidimetric immunoassay (TIA), enzyme immunoassay (EIA) (e.g., direct competitive ELISA, indirect competitive ELISA, and sandwich ELISA), radioimmunoassay (RIA), latex agglutination reaction method, fluorescence immunoassay (FIA), and immunochromatography, Western blotting, immunostaining and fluorescence activated cell sorting (FACS). In the case of detecting multiple components, proteomic analysis may be performed.

In the case that the component to be analyzed is a nucleic acid, examples of the analysis method include hybridization methods using probes, reverse transcription (RT) reactions using reverse transcriptase, nucleic acid amplification methods (e.g., PCR methods such as quantitative PCR, RT-PCR) using primer (e.g., 2, 3 or 4 primers), sequencing and mass spectrometry.

In the case that the component to be analyzed is a component other than a protein or a nucleic acid, examples of the analysis method include immunoassay and mass spectrometry. In the case of analyzing multiple components, metabolome analysis can be performed.

The analysis method of the present invention can be used for detection of the marker contained in the extracellular vesicle. Examples of the marker contained in the extracellular vesicle include the EV markers (e.g., EV inside marker and EV surface marker) described above.

Analysis of the extracellular vesicle itself (particle) can be performed with equipment such as particle analysis equipment, electron microscope and flow cytometer, for example. In this case, it is possible to analyze the number, dimension, shape of particles of the extracellular vesicle and distribution thereof.

The analysis method of the present invention may further include disrupting the extracellular vesicle obtained in the present invention when the EV inside marker is analyzed, for example. In such a case, the analysis method of the present invention is performed by the following steps:
(1) disrupting the extracellular vesicle obtained in the present invention; and
(2) detecting the extracellular vesicle inside marker of the disrupted extracellular vesicle.

The disruption of the extracellular vesicle may be disruption by treatment with chemical substance, disruption by physical operation treatment, or a combination thereof, for example. Examples of the chemical substances include an extracellular vesicle-disruptive substance. As the extracellular vesicle-disruptive substance, it is possible to use the EV-disruptive surfactant described above, for example. The EV-disruptive surfactant used for the disruption of the extracellular vesicle is preferably a surfactant other than the EV-washable nonionic surfactants (e.g., alcohol ethoxylate, polyoxyethylene-polyoxypropylene block copolymer) described above, and more preferably a nonionic surfactant having an HLB less than 15 or an ionic surfactant having the number of carbon atoms of 11 or more in the carbon chain. Examples of the physical operation include pressurization.

### 5. Kit

The present invention also provides a kit that includes as follows:
(1) the EV-washable nonionic surfactant; and
(2) the extracellular vesicle membrane-binding substance.

The kit of the present invention may further include (3) the extracellular vesicle inside marker binding substance. The kit of the present invention may further include (4) the EV-disruptive surfactant.

The EV-washable nonionic surfactant, the extracellular vesicle membrane-binding substance, the extracellular vesicle inside marker-binding substance and the EV-disruptive surfactant that are included in the kit of the present invention, are described above. The kit of the present invention is useful in the convenient practice of the washing method of the present invention, the recovery method of the present invention, the production method of the present invention, and the analysis method of the present invention, for example.

### EXAMPLES

Hereinafter, the present invention will be described with reference to Examples, but the present invention is not limited to these Examples.

(Example 1: Preparation of solid-phase antibody and labeled antibody, and a method of measuring EV surface marker)

### (1) Preparation of antibody solid-phased particles (solid-phase antibody)

To 0.01 g/mL of magnetic particles in 10 mM MES buffer (pH 5.0), 0.2 mg/mL of one of a mouse anti-CD9 monoclonal antibody (Fujirebio Inc.), a mouse anti-CD63 monoclonal antibody (Fujirebio Inc.) and a mouse anti-CD81 monoclonal antibody (Fujirebio Inc.) that recognize extracellular vesicle (EV) surface markers, CD9, CD63 and CD81, respectively, was added. Then, the resulting solution was gently stirred at 25°C for one hour for incubation. After the reaction, the magnetic particles were magnetically collected and washed with a washing solution (50 mM Tris buffer, 150 mM NaCl₂, 2.0% BSA, pH 7.2) in order to obtain anti-CD9 antibody solid-phased particles, anti-CD63 antibody solid-phased particles and anti-CD81 antibody solid-phased particles. These antibody solid-phased particles were suspended in a particle diluent (50 mM Tris buffer, 1 mM EDTA·2Na, 0.1% NaN₃, 2.0% BSA, pH 7.2) in order to obtain each antibody solid-phased particle solution.

### (2) Preparation of alkaline phosphatase labeled antibody

Desalted alkaline phosphatase (ALP) was mixed with N-(4-maleimidobutyryloxy)-succinimide (GMBS) (final concentration 0.3 mg/mL) and the resulting solution was left to stand at 30°C for one hour for maleimidation. Next, in the coupling reactant solution (100 mM phosphate buffer, 1 mM EDTA·2Na, pH 6.3), one of Fab's of mouse anti-CD9 monoclonal antibody, mouse anti-CD63 monoclonal antibody and mouse anti-CD81 monoclonal antibody was mixed with maleimidized ALP at a molar ratio of 1: 1, and reacted at 25°C for one hour. The reactant solution was subjected to purification using Superdex 200 column chromatography (General electric company) to obtain ALP-labeled anti-CD9 antibody, ALP-labeled anti-CD63 antibody and ALP-labeled anti-CD81 antibody. Each ALP-labeled antibody was suspended in a labeled antibody diluent (50 mM MES buffer, 150 mM NaCl₂, 0.3 mM ZnCl₂, 1 mM MgCl₂, 0.1% NaN₃, 2.0% BSA, pH 6.8) to obtain each labeled antibody solution.

### (3) Measurement method for EV surface marker (CD9, CD63 and CD81)

The measurement method for EV surface marker (CD9, CD63 and CD81) in the following examples is as follows.

20 µL of the sample to be measured was dispensed into a reaction vessel, and 50 µL of anti-CD9 antibody solid-phased particles solution, anti-CD63 antibody solid-phased particles solution or anti-CD81 antibody solid-phased particles solution was dispensed and then stirred. Then, the resulting solution was incubated at 37°C for 8 minutes, and B/F separation and washing were carried out. 50 µL of the ALP-labeled antibody corresponding to the solid-phase antibody was dispensed into the reaction vessel, the resulting solution was stirred and then incubated at 37°C for 8 minutes, and B/F separation and washing were carried out. Then, 200 µL of Lumipulse (registered trademark) substrate solution (Fujirebio Inc.) containing 3-(2'-spiroadamantane)-4-methoxy-4-(3''-phosphoryloxy)phenyl-1,2-dioxetane disodium salt (AMPPD) serving as a chemiluminescent substance was dispensed into the reaction vessel. The resulting solution was stirred and then incubated at 37°C for 4 minutes for the measurement of luminescence intensity using luminometer. The measurement was actually carried out using a fully automated chemiluminescent enzyme immunoassay system (Lumipulse L2400 (Fujirebio Inc.)).

Antibodies recognizing the same epitope were used as the antibody solid-phased on the magnetic particle and the ALP-labeled antibody. Due to the presence of a plurality of CD9, CD63 or CD81 on a single extracellular vesicle, it is possible to measure for the extracellular vesicle and the markers thereof by the sandwich immunoassay in which the antibodies recognizing the same epitope as the solid-phase antibodies were used as the ALP-labeled antibodies.

### (Example 2: Screening of EV-nondisruptive surfactant and EV-disruptive surfactant based on EV surface marker measurement)

The influence of various surfactants on the extracellular vesicle with the EV marker used as an indicator was measured for the screening of the EV-nondisruptive surfactant and EV-disruptive surfactant.

To 500 µL of human serum specimen, 250 µL of EDTA and EGTA-containing PBS (EDTA/EGTA/PBS) containing 0.1 w/v% of each surfactant was added (each of final concentrations of EDTA and EGTA is 50 mM after the mixing with the serum specimen, pH 7.4). The mixture solution was rotated for reaction at 37°C for 30 minutes, and then used as a sample to be measured. Then, reactivity (amount measured for the EV surface marker) of CD9, CD63 or CD81 in the sample to be measured was measured according to the measurement method of Example 1 (3). As a control, EDTA/EGTA/PBS not containing the surfactant was used. Based on obtained count values, the ratio (influence ratio %) of the count value with the addition of each surfactant was determined with reference to the count value of control.

Results are listed in Table 2. Compared to the control, the reduction of the reactivity by 20% or more (the influence ratio less than 80%) is interpreted to cause disruption of the extracellular vesicle, and the surfactant used is interpreted as an EV disruptive surfactant.. In the case with the reduction of the reactivity by less than 20% (the influence ratio of 80% or more), the surfactant used is interpreted as a surfactant not influencing on the extracellular vesicle (EV-nondisruptive surfactant).

**Table 2. Screening of EV-disruptive surfactants and EV-nondisruptive surfactants based on EV disruptive property when the influence on amount measured for the EV surface marker is used as an indicator**

| | Influence ratio % | | | Physical property value and the like of compound | | |
|---|---|---|---|---|---|---|
| | CD9 | CD63 | CD81 | Hydrophobic side chain* | HLB | CAS No. |
| Nonionic surfactants | | | | | | |
| Tergitol 15-S-7 | | 15.3 | 2.2 | 13 | 12.1 | 84133-50-6 |
| Tergitol 15-S-9 | 11.1 | 26.2 | 10.9 | 13 | 13.3 | 84133-50-6 |
| Tergitol 15-S-30 | 113.0 | 96.1 | 102.4 | 13 | 17.4 | 68131-40-8 |
| Tergitol 15-S-40 | 8.9 105.8 | 91.0 | 103.4 | 13 | 18 | 68131-40-8 |
| Brij 58 | 131.3 | 87.0 | 86.9 | 15 | 15.7 | 9004-95-9 |
| Brij 35 | 130.7 | 98.5 | 102.3 | 12 | 16 | 9002-92-0 |
| Pluronic F68 | 99.8 | 95.7 | 118.9 | (Block copolymer) | >24** | 9003-11-6 |
| Pluronic F127 | 94.5 | 91.5 | 104.3 | (Block copolymer) | 18-23** | 9003-11-6 |
| TWEEN20 | 151.6 | 98.3 | 84.2 | 11 | 16.7 | 9005-64-5 |
| TWEEN40 | 103.7 | 88.5 | 82.0 | 15 | 15.6 | 9005-66-7 |
| TWEEN80 | 114.6 | 92.2 | 95.7 | 17 | 15 | 9005-65-6 |
| Triton X-100 | 9.5 | 16.1 | 9. | Octylphenyl ether | 13.5 | 9002-93-1 |
| Triton X-114 | 17.7 | 40.4 | 10.2 | Octylphenyl ether | 12.4 | 9036-19-5 |
| Triton X-305 | 111.1 | 98.0 | 119.5 | Octylphenyl ether | 17.3 | 9002-93-1 |
| Triton X-405 | 111.0 | 100.5 | 115.9 | Octylphenyl ether | 17.6 | 9036-19-5 |
| Triton X-705 | 121.6 | 94.3 | 103.5 | Octylphenyl ether | 18.4 | 9036-19-5 |
| MEGA 8 | 96.6 | 99.9 | 105.7 | 7 | 8.6 | 85316-98-9 |
| MEGA 10 | 105.2 | 89.4 | 87.5 | 9 | 8.4 | 85261-20-7 |

| Cationic surfactants | | | | | | |
|---|---|---|---|---|---|---|
| C8TAB | 99.9 | 87.9 | 94.4 | 8 | | 2083-68-3 |
| C9TAB | 101.4 | 93.1 | 108.7 | 9 | | 1943-11-9 |
| C12TAB | 85.8 | 66.2 | 78.8 | 12 | | 1119-94-4 |
| C14TAB | 2.4 | 2.7 | 0.5 | 14 | | 1119-97-7 |
| C16TAB | 3.3 | 1.7 | 10.4 | 16 | | 57-09-0 |
| C12TAC | 83.0 | 35.0 | 21.4 | 12 | | 112-00-5 |
| C14TAC | 1.7 | 1.9 | 10.3 | 14 | | 4574-04-3 |
| C16TAC | 2.6 | 1.9 | 11.7 | 16 | | 112-02-7 |
| C18TAC | 5.3 | 2.1 | 4.2 | 18 | | 112-03-8 |

| | Influence ratio % | | | Physical property value and the like of compound | | |
|---|---|---|---|---|---|---|
| | CD9 | CD63 | CD81 | Hydrophobic side chain* | HLB | CAS No. |
| Anionic surfactants | | | | | | |
| NDS | 100.0 | 95.7 | 81.7 | 9 | | 30377-07-2 |
| NLS | 36.2 | 30.8 | 19.5 | 11 | | 137-16-6 |
| NSS | 95.2 | 90.6 | 90.0 | 9 | | 35192-74-6 |
| CSSS | 101.9 | 87.4 | 106.4 | | | 9082-07-9 |
| SDBS | 2.8 | 2.6 | 13.9 | 12 | | 25155-30-0 |

| Zwitterionic surfactant | | | | | | |
|---|---|---|---|---|---|---|
| CHAPS | 117.3 | 94.2 | 123.4 | | | 75621-03-3 |
| C8APS | 91.1 | 95.9 | 100.2 | 8 | | 15178-76-4 |
| C10APS | 118.8 | 89.5 | 100.9 10 | | | 15163-36-7 |
| C12APS | 15.1 | 15.5 | 12.2 | 12 | | 14933-08-5 |
| C14APS | 2.8 | 2.8 | 10.7 | 14 | | 14933-09-6 |
| C16APS | 4.3 | 3.2 | 6.5 | 16 | | 2281-11-0 |
| NDSB-195 | 99.6 | 89.7 | 91.3 | 2 | | 160255-06-1 |
| NDSB-211 | 100.0 | 90.7 | 109.1 | Propanesulfonic acid | | 38880-58-9 |
| NDSB256 | 91.4 | 85.8 | 115.8 | Benzyldimethyl | | 81239-45-4 |

| | | | | | | |
|---|---|---|---|---|---|---|
| *Number indicates the number of the carbon atoms when hydrophobic side chain is alkyl chain. **The value described in the manufacturer's data sheet. The vale exceed 20. | | | | | | |

This result demonstrates that a nonionic surfactant with an HLB of 15 or more and a linear ionic surfactant with the number of carbon atoms of 10 or less in a carbon chain tend to be the surfactants not disrupting the extracellular vesicle (EV-nondisruptive surfactants). These surfactants can be candidates for the surfactants for washing the extracellular vesicle. Meanwhile, it demonstrates that a nonionic surfactant with an HLB less than 15 and an ionic surfactant with the number of carbon atoms of 11 or more in a carbon chain tend to be the surfactants efficiently disrupting EV (EV-disruptive surfactants). These surfactants can be used for disrupting the extracellular vesicle.

### (Example 3: Investigation of EV disruptive property and EV nondisruptive property dependent on concentration of surfactant based on measurement for EV inside marker)

The concentration dependent influence of various surfactants on disruptive and nondisruptive properties for EV with the EV inside marker used as the indicator was measured.

A culture supernatant of human colon carcinoma cell line DU-145 cultured in a serum-free medium for three days was centrifuged at 2,000 × g at 4°C for 5 minutes, then filtered through the 0.22 µm filter (manufactured by Millipore Corp.), and then concentrated using Amicon Ultra-15 (manufactured by Millipore Corp.). The concentrate was centrifuged at 20,000 × g at 4°C for 15 minutes. Next, the supernatant was centrifuged at 100,000 × g at 4°C for one hour. The supernatant was discarded, and then PBS (pH 7.4) or 50 mM EDTA/50 mM EGTA/PBS (PBS containing 50 mM EDTA and 50 mM EGTA, pH 7.4) was added to resuspend the precipitate. Then, the resuspension was centrifuged at 150,000 × g at 4°C for one hour. The supernatant was discarded, and PBS or 50 mM EDTA/50 mM EGTA/PBS was newly added to resuspend the precipitate in order to obtain EV (DU-145). The concentration of the EV (DU-145) was performed with Qubit Protein Assay (Thermo Fisher Scientific Inc.). 10 µL of 63 µg/mL EV (DU-145) was mixed with 10 µL of the surfactant solution (see Table for concentration). After the reaction at room temperature for 30 minutes, 190 µL of Lumipulse specimen diluent (Fujirebio Inc.) was added. The measurement was carried out using Lumipulse L2400 (Fujirebio Inc.) for the EV inside markers (CEA and CA125) contained in the sample with use of Lumipulse Presto CEA reagent (Fujirebio Inc.) and Lumipulse Presto CA125-II reagent (Fujirebio Inc.) according to an attached protocol.

Results are listed in Table 3. The increase in the reactivity (amount measured for the EV surface marker) by 20% or more compared to the case without addition of surfactant, is interpreted to cause disruption of membrane of EV (DU-145) and discharge of the EV inside antigens (CEA and CA125), and the surfactant used is interpreted as an EV disruptive surfactant.

**Table 3. Investigation of EV disruptive property and EV nondisruptive property dependent on concentration of surfactant**

| | | CEA | | | | CA125 | | | |
|---|---|---|---|---|---|---|---|---|---|
| Count | Without addition | 855 | | | | 5690 | | | |

| Concentration of surfactant | | 0.01% | 0.10% | 0.50% | 2% | 0.01% | 0.10% | 0.50% | 2% |
|---|---|---|---|---|---|---|---|---|---|
| Count | Tween20 | 1058 | 1522 | 1725 | 1622 | 7358 | 9075 | 10669 | 10643 |
| | Tween80 | 934 | 1000 | 1097 | 1144 | 6098 | 6413 | 7815 | 8629 |
| | Triton X-100 | 980 | 1812 | 1941 | 1993 | 7030 | 11533 | 11429 | 12427 |
| | Tergitol 15 S-9 | 1283 | 1879 | 1617 | 1845 | 9666 | 11178 | 11286 | 12471 |
| | Tergitol 15-S-30 | 938 | 959 | 987 | 992 | 5878 | 6213 | 6394 | 6646 |
| | Tergitol 15-S-40 | 912 | 997 | 967 | 959 | 6047 | 6008 | 6290 | 6492 |
| | Pluronic F68 | 982 | 948 | 916 | 908 | 5840 | 5852 | 6285 | 6514 |
| | Pluronic F127 | 870 | 998 | 950 | 955 | 6026 | 5954 | 6116 | 6157 |
| | C8TAB | 927 | 953 | 936 | 964 | 5696 | 5738 | 5751 | 6021 |
| Against Without addition % | Tween20 | 123.7 | 178 | 201.8 | 189.7 | 129.3 | 159.5 | 187.5 | 187 |
| | Tween80 | 109.2 | 117 | 128.3 | 133.8 | 107.2 | 112.7 | 137.3 | 151.7 |
| | Triton X-100 | 114.6 | 211.9 | 227 | 233.1 | 123.6 | 202.7 | 200.9 | 218.4 |
| | Tergitol 15 S-9 | 150.1 | 219.8 | 189.1 | 215.8 | 169.9 | 196.4 | 198.3 | 219.2 |
| | Tergitol 15-S-30 | 109.7 | 112.2 | 115.4 | 116 | 103.3 | 109.2 | 112.4 | 116.8 |
| | Tergitol 15-S-40 | 106.7 | 116.6 | 113.1 | 112.2 | 106.3 | 105.6 | 110.5 | 114.1 |
| | Pluronic F68 | 114.9 | 110.9 | 107.1 | 106.2 | 102.6 | 102.8 | 110.5 | 114.5 |
| | Pluronic F127 | 101.8 | 116.7 | 111.1 | 111.7 | 105.9 | 104.6 | 107.5 | 108.2 |
| | C8TAB | 108.4 | 111.5 | 109.5 | 112.7 | 100.1 | 100.8 | 101.1 | 105.8 |

This result reveals that, among the nonionic surfactants with HLB of 15 or more, Tergitols, Pluronics and C8TAB, which is a linear ionic surfactant with the number of carbon atoms of 10 or less in a carbon chain, are the surfactants without causing disruption of EV (DU-145) and discharge of antigens in the EV (EV-nondisruptive surfactant). Meanwhile, it reveals that, among the nonionic surfactants with HLB of 15 or more, Tween (registered trademark) 20 and Tween 80 have effects of causing disruption of EV (DU-145) (EV-disruptive properties).

### (Example 4: Investigation of inhibitory effect of nonionic surfactant in EV operation system on absorption of specimen component to container)

Inhibitory effect of the nonionic surfactant in the EV operation system on absorption of specimen component to container was investigated in terms of the total amount of residual proteins as an indicator.

In a polypropylene 1.5 mL tube (QSP Inc.) (referred to as "reaction tube", hereinafter), 250 µL of EDTA/EGTA/PBS containing 1.5 w/v% of each nonionic surfactant was added to 500 µL of human serum specimen. The surfactants used were Tween 20, Triton-X100, Tergitol 15-s-30, Brij35, Brij58, Pluronic F68, C8TAB, NSS, C8APS, NDSB-195, and NDSB-256. After the reactant solution was rotated for reaction at 37°C for 30 minutes, and then the resulting solution was removed from the reaction tube. After the solution was removed, the reaction tube was washed 5 times with PBS used as a washing solution. After each time of the washing, the resultant washing solution was transferred to individual new tubes and labeled as "washing fractions 1 to 5". The empty reaction tubes obtained after 5 times washing are referred as "containers obtained after reaction". The containers obtained after reaction and the washing fractions were used for BCA assay (Thermo Fisher Scientific Inc.). To the containers obtained after reaction, 1 mL of BCA solution was added for reaction at 37°C for 30 minutes. To 20 µL of washing fractions 1 to 5, 200 µL of the BCA solution was added for reaction at 37°C for 30 minutes. After the reactions, an absorbance at 562 nm was measured using a microplate reader (Tecan Group Ltd.) for determination of the total amount of proteins.

Results are listed in Table 4. With the addition, all of the investigated nonionic surfactants decrease the total amount of proteins dissolved in the washing fractions, and decrease the total amount of proteins left in the containers.

**Table 4. Investigation of inhibitory effect of various surfactants on absorption of specimen component to container**

| Surfactant | | Without addition | Tween20 | Triton X-100 | Tergitol 15-S-30 | Brij35 | Brij58 |
|---|---|---|---|---|---|---|---|
| Type of the Surfactant | | | Nonion ic | Nonion ic | Nonion ic | Nonion ic | Nonion ic |
| A562nm | Washing fraction 1 | 0.5989 | 0.3704 | 0.622 | 0.1621 | 0.2617 | 0.3019 |
| | Washing fraction 2 | 0.1181 | 0.1065 | 0.1073 | 0.1031 | 0.1063 | 0.108 |
| | Washing fraction 3 | 0.1137 | 0.1025 | 0.102 | 0.1018 | 0.1054 | 0.1059 |
| | Washing fraction 4 | 0.1032 | 0.1025 | 0.1011 | 0.1016 | 0.1047 | 0.1035 |
| | Washing fraction 5 | 0.1067 | 0.1022 | 0.1006 | 0.1008 | 0.1061 | 0.1032 |
| | Containers after immunoprecipitation | 0.1419 | 0.124 | 0.1207 | 0.124 | 0.1285 | 0.1253 |
| Against Without addition % | Washing fraction 1 | | 61.8 | 103.9 | 27.1 | 43.7 | 50.4 |
| | Washing fraction 2 | | 90.2 | 90.9 | 87.3 | 90 | 91.4 |
| | Washing fraction 3 | | 90.1 | 89.7 | 89.5 | 92.7 | 93.1 |
| | Washing fraction 4 | | 99.3 | 98 | 98.4 | 101.5 | 100.3 |
| | Washing fraction 5 | | 95.8 | 94.3 | 94.5 | 99.4 | 96.7 |
| | Containers after immunoprecipitation | | 87.4 | 85.1 | 87.4 | 90.6 | 88.3 |
| | | | | | | | |

| Surfactant | | Pluronic F68 | C8TAB | NSS | C8APS | NDSB-195 | NDSB -256 |
|---|---|---|---|---|---|---|---|
| Type of the Surfactant | | Nonion ic | Cation ic | Anioni c | Zwitte rionic | Zwitte rionic | Zwitte rionic |
| A562nm | Washing fraction 1 | 0.1796 | 0.6486 | 0.5855 | 0.5838 | 0.6625 | 0.6015 |
| | Washing fraction 2 | 0.1014 | 0.1184 | 0.1149 | 0.1168 | 0.1186 | 0.1148 |
| | Washing fraction 3 | 0.1013 | 0.103 | 0.1055 | 0.1047 | 0.1069 | 0.1018 |
| | Washing fraction 4 | 0.1002 | 0.1028 | 0.1034 | 0.1036 | 0.103 | 0.1022 |
| | Washing fraction 5 | 0.1007 | 0.1026 | 0.1041 | 0.1038 | 0.1045 | 0.1022 |
| | Containers after immunoprecipitation | 0.1247 | 0.2809 | 0.2082 | 0.2195 | 0.1569 | 0.202 |
| Against Without addition % | Washing fraction 1 | 30 | 108.3 | 97.8 | 97.5 | 110.6 | 100.4 |
| | Washing fraction 2 | 85.9 | 100.3 | 97.3 | 98.9 | 100.4 | 97.2 |
| | Washing fraction 3 | 89.1 | 90.6 | 92.8 | 92.1 | 94 | 89.5 |
| | Washing fraction 4 | 97.1 | 99.6 | 100.2 | 100.4 | 99.8 | 99 |
| | Washing fraction 5 | 94.4 | 96.2 | 97.6 | 97.3 | 97.9 | 95.8 |
| | Containers after immunoprecipitation | 87.9 | 198.0 | 146.7 | 154.7 | 110.6 | 142.4 |

This result reveals that the addition of the nonionic surfactant in the reactant solution enables reduction of contamination with impurities into the obtained EV sample.

### (Example 5: Investigation of inhibitory effect of nonionic surfactant in EV recovery by immunoprecipitation on absorption of specimen component to container)

Washing effect of the nonionic surfactant in EV recovery by immunoprecipitation on absorption of specimen component to container was investigated in terms of the total amount of residual proteins as an indicator.

In the polypropylene 1.5 mL tube (QSP Inc.) (referred to as "reaction tube", hereinafter), to 500 µL of human serum specimen, 250 µL of EDTA/EGTA/PBS was added and then the anti-CD9 antibody solid-phased particles were added at 0.15 w/v%. The reactant solution was rotated for reaction at 37°C for 30 minutes in the reaction tube, and then magnetically collected the particles for collection of the supernatant. PBS containing 0.05 w/v% of each surfactant was used as the washing solution for washing 5 times the magnetic particles after the reaction. The same surfactants were used as in Example 4. After each time of the washing, the resultant washing solution was transferred to individual new tubes and labeled as "washing fractions 1 to 5". The washed magnetic particles were transferred to other new tubes. The reaction tubes were washed once with the washing solution under each condition, and labeled as "containers after immunoprecipitation". The containers after immunoprecipitation and the washing fractions 1 to 5 were used for BCA assay (Thermo Fisher Scientific Inc.). To the containers after immunoprecipitation, 1 mL of BCA solution was added for reaction at 37°C for 30 minutes. To 20 µL of the washing fractions, 200 µL of the BCA solution was added for reaction at 37°C for 30 minutes. After the reactions, the absorbance at 562 nm was measured using the microplate reader (Tecan Group Ltd.) for determination of the total amount of proteins.

Results are listed in Table 5. With the addition to the washing solution, all of the investigated nonionic surfactants tend to increase the total amount of proteins eluded in the washing fractions, decreasing the total amount of proteins left in the containers. In particular, Tween 20, TritonX-100, Tertitol 15-S-30, Brij35, Brij58 exhibited high washing effects.

**Table 5. Investigation of effect when washing of various surfactants**

| Nonionic surfactant | | Without addition | Tween20 | Triton X-100 | Tergitol 15-S-30 | Brij35 | Brij58 |
|---|---|---|---|---|---|---|---|
| Type of the Surfactant | | | Nonion ic | Nonion ic | Nonion ic | Nonion ic | Nonion ic |
| A562nm | Washing fraction 1 | 2.2755 | 2.5606 | 2.7655 | 2.2342 | 2.4512 | 2.3275 |
| | Washing fraction 2 | 0.2141 | 0.1901 | 0.2208 | 0.2169 | 0.2235 | 0.2057 |
| | Washing fraction 3 | 0.1268 | 0.1236 | 0.1401 | 0.1241 | 0.1439 | 0.1338 |
| | Washing fraction 4 | 0.1166 | 0.1155 | 0.1138 | 0.1156 | 0.1397 | 0.1267 |
| | Washing fraction 5 | 0.1122 | 0.1126 | 0.1127 | 0.1166 | 0.1425 | 0.1295 |
| | Containers after immunoprecipitation | 0.3192 | 0.1302 | 0.1193 | 0.1483 | 0.1923 | 0.1461 |
| Against Without addition % | Washing fraction 1 | | 112.5 | 121.5 | 98.2 | 107.7 | 102.3 |
| | Washing fraction 2 | | 88.8 | 103.1 | 101.3 | 104.4 | 96.1 |
| | Washing fraction 3 | | 97.5 | 110.5 | 97.9 | 113.5 | 105.5 |
| | Washing fraction 4 | | 99.1 | 97.6 | 99.1 | 119.8 | 108.7 |
| | Washing fraction 5 | | 100.4 | 100.4 | 103.9 | 127 | 115.4 |
| | Containers after immunoprecipitation | | 40.8 | 37.4 | 46.5 | 60.2 | 45.8 |

| Nonionic surfactant | | Pluronic F68 | C8TAB | NSS | C8APS | NDSB- 195 | NDSB-256 |
|---|---|---|---|---|---|---|---|
| Type of the Surfactant | | Nonionic | Cationic | Anionic | Zwitte rionic | Zwitte rionic | Zwitte rionic |
| A562nm | Washing fraction 1 | 2.3906 | 2.4782 | 2.9322 | 2.8302 | 2.4661 | 2.5691 |
| | Washing fraction 2 | 0.2744 | | 0.2596 | 0.2785 | 0.2427 | 0.2442 |
| | Washing fraction 3 | 0.1269 | 0.2045 0.1211 | 0.1222 | 0.1185 | 0.1169 | 0.1163 |
| | Washing fraction 4 | 0.1156 | 0.1158 | 0.116 | 0.1102 | 0.1119 | 0.1119 |
| | Washing fraction 5 | 0.1128 | 0.1131 | 0.113 | 0.1132 | 0.1093 | 0.1136 |
| | Containers after immunoprecipitation | 0.2338 | 0.3436 | 0.3536 | 0.332 | 0.3136 | 0.3437 |
| Against Without addition % | Washing fraction 1 | 105.1 | 108.9 | 128.9 | 124.4 | 108.4 | 112.9 |
| | Washing fraction 2 | 128.2 | 95.5 | 121.3 | 130.1 | 113.4 | 114.1 |
| | Washing fraction 3 | 100.1 | 95.5 | 96.4 | 93.5 | 92.2 | 91.7 |
| | Washing fraction 4 | 99.1 | 99.3 | 99.5 | 94.5 | 96 | 96 |
| | Washing fraction 5 | 100.5 | 100.8 | 100.7 | 100.9 | 97.4 | 101.2 |
| | Containers after immunoprecipitation | 73.2 | 107.6 | 110.8 | 104 | 98.2 | 107.7 |

This result reveals that the washing with the addition of the nonionic surfactant reduces contamination with impurities into the obtained EV sample.

(Example 6: Investigation of concentration dependence of washing effect of nonionic surfactant in EV recovery by immunoprecipitation)

Concentration dependence of the washing effect of the nonionic surfactant in EV recovery by immunoprecipitation was investigated in terms of the total amount of residual proteins as an indicator.

In the polypropylene 1.5 mL tube (QSP Inc.) (referred to as "reaction tube", hereinafter), to 500 µL of human serum specimen, 250 µL of EDTA/EGTA/PBS was added and then the anti-CD9 antibody solid-phased particles were added at 0.15 w/v%. The reactant solution was rotated for reaction at 37°C for 30 minutes in the reaction tube, and then magnetically collected. Then, the resulting supernatant was transferred to another new tube. The magnetic particles obtained after the reaction was washed 5 times with PBS containing different concentrations (0 w/v%, 0.01 w/v%, 0.05 w/v%, 0.1 w/v%, 0.25 w/v%, 0.5 w/v% and 1.0 w/v%) of Tergitol 15-s-30 or Pluronic F68 used as the washing solution. The magnetic particles obtained after the washing was transferred to another new tube. The reaction tube was washed once with the washing solution under each condition, and labeled as "container obtained after immunoprecipitation". The container obtained after immunoprecipitation was used for BCA assay (Thermo Fisher Scientific Inc.). To the containers obtained after immunoprecipitation, 1 mL of BCA solution was added for reaction at 37°C for 30 minutes. After the reaction, the absorbance at 562 nm was measured using the microplate reader (Tecan Group Ltd.) for determination of the total amount of proteins.

Results are given in Table 6 and Fig. 1. With the addition of Tergitol 15-s-30 or pluronic F68 at 0.01% or more into the washing solution, the amount of the specimen component (residual total proteins) absorbed to the container was reduced so as to exhibit the washing effect. In particular, Tergitol 15-s-30 exhibit superior washing effect.

**Table 6. Comparison of concentration-dependent washing effect of Tergitol and Pluronic**

| Addition concentration of surfactant (%) | | Without addition | 0.01 | 0.05 | 0.1 | 0.25 | 0.5 | 1 |
|---|---|---|---|---|---|---|---|---|
| A562nm | Tergitol15-S-30 | 0.4153 | 0.1842 | 0.1442 | 0.1506 | 0.1504 | 0.1417 | 0.166 |
| | PluronicF68 | 0.3844 | 0.2646 | 0.2151 | 0.2046 | 0.2358 | 0.1989 | 0.2129 |
| Against Without addition (%) | Tergitol15-S-30 | | 44.4 | 34.7 | 36.3 | 36.2 | 34.1 | 40 |
| | PluronicF68 | | 68.8 | 56 | 53.2 | 61.3 | 51.7 | 55.4 |

This result exhibits that the washing with the addition of Tergitol 15-s-30 further reduces the contamination with impurities into the obtained EV sample.

### (Example 7: Investigation of washing effect in EV recovery from specimen)

Washing effect in the EV recovery from the specimen was investigated.

In the polypropylene 1.5 mL tube (QSP Inc.) (referred to as "reaction tube", hereinafter), to 500 µL of human serum specimen, 250 µL of EDTA/EGTA/PBS was added and then the anti-CD9 antibody solid-phased particles were added at 0.15 w/v%. The reactant solution was rotated for reaction at 37°C for 30 minutes in the reaction tube, and then magnetically collected. Then, the resulting supernatant was transferred to another new tube, and labeled as "SUP sample". The magnetic particles obtained after the reaction was washed 5 times with PBS (pH 7.4) or PBS containing Tergitol 15-s-30 (0.05 w/v% Tergitol 15-s-30, pH 7.4) used as the washing solution. Each washing solution obtained after each washing was transferred to an individual new tube, and labeled as "washing fractions 1 to 5". The magnetic particles obtained after the washing was transferred to another new tube, and labeled as "particles obtained after immunoprecipitation". The reaction tube was washed once with the washing solution under each condition, and labeled as "container obtained after immunoprecipitation". The particles obtained after immunoprecipitation, the container obtained after immunoprecipitation and the washing fractions were used for BCA assay (Thermo Fisher Scientific Inc.). To each of the particles obtained after immunoprecipitation and the container obtained after immunoprecipitation, 1 mL of BCA solution was added for reaction at 37°C for 30 minutes. To 20 µL of each of the washing fractions 1 to 5, 200 µL of the BCA solution was added for reaction at 37°C for 30 minutes. After the reactions, the absorbance at 562 nm was measured using the microplate reader (Tecan Group Ltd.) for determination of the total amount of proteins. For the SUP sample and the washing fractions, a CEA amount was measured using Lumipulse L2400 (Fujirebio Inc.) with use of Lumipulse Presto CEA reagent (Fujirebio Inc.).

Table 7 represents results of the total amount of proteins measured by the BCA assay. Table 8 represents results of the CEA amount measurement. The addition of Tergitol 15-s-30 into the washing solution increases the total amount of proteins eluted in the washing fraction compared to the case without addition, and decreases the total amount of proteins left in the container to 62.3% after the immunoprecipitation. Also, the addition of Tergitol 15-s-30 into the washing solution increases the amount of CEA eluted in the washing fraction and decreases the amount of CEA left in the container.

**Table 7. Confirmation of washing effect in total amount of proteins measurement**

| BCA assay | | | |
|---|---|---|---|
| Tergitol in washing solution | | - | + |
| A562nm | Washing fraction 1 | 1.3587 | 1.6374 |
| | Washing fraction 2 | 0.1668 | 0.1829 |
| | Washing fraction 3 | 0.109 | 0.1101 |
| | Washing fraction 4 | 0.1071 | 0.1069 |
| | Washing fraction 5 | 0.1081 | 0.1053 |
| | Containers after immunoprecipitation | 0.2325 | 0.1448 |
| | Particles after immunoprecipitation | 0.9586 | 0.9754 |
| Against Without addition % | Washing fraction 1 | | 120.5 |
| | Washing fraction 2 | | 109.7 |
| | Washing fraction 3 | | 101 |
| | Washing fraction 4 | | 99.8 |
| | Washing fraction 5 | | 97.4 |
| | Containers after immunoprecipitation | | 62.3 |
| | Particles after immunoprecipitation | | 101.8 |

**Table 8. Confirmation of washing effect in CEA amount measurement**

| CEA assay | | | |
|---|---|---|---|
| Tergitol in washing solution | | - | + |
| Count | SUP | 8197677 | 8130501 |
| | Washing fraction 1 | 273848 | 371759 |
| | Washing fraction 2 | 9993 | 11175 |
| | Washing fraction 3 | 1264 | 1203 |
| | Washing fraction 4 | 960 | 1020 |
| | Washing fraction 5 | 1132 | 984 |
| Against Without addition % | SUP | | 99.2 |
| | Washing fraction 1 | | 135.8 |
| | Washing fraction 2 | | 111.8 |
| | Washing fraction 3 | | 95.2 86.9 |
| | Washing fraction 4 | | 106.3 |
| | Washing fraction 5 | | |

This result reveals that the washing with the addition of Tergitol 15-s-30 reduces the contamination with impurities into the obtained EV sample.

### (Example 8: Confirmation of washing effect in EV marker measurement)

EV was recovered from the specimen, and disrupted for the measurement for the marker in the EV.

In the polypropylene 1.5 mL tube (QSP Inc.) (referred to as "reaction tube", hereinafter), to 500 µL of human serum specimen, 250 µL of EDTA/EGTA/PBS was added. The resulting solution was labeled as "original solution sample". To the original solution sample, anti-CD9 antibody solid-phased particles, anti-CD63 antibody solid-phased particles, anti-CD81 antibody solid-phased particles or a mixture thereof was added at 0.15 w/v%. The reactant solution was rotated for reaction at 37°C for 30 minutes in the reaction tube, and then magnetically collected. Then, the resulting supernatant was transferred to another new tube, and labeled as "SUP sample". The magnetic particles obtained after the reaction was washed 5 times with PBS containing 0.05% of Tergitol 15-S-30 used as the washing solution. Each washing solution obtained after each washing was transferred to an individual new tube, and labeled as "washing fractions 1 to 5". To the container (reaction tube) that contains the magnetic particles obtained after the washing , 20 µL of Lysis buffer (D-PBS (-), 150 mM NaCl, 1.0% N-lauroyl sarcosine sodium, 2.0% N-dodecyl-N,N-dimethyl-3-ammonio-1-propane sulfonate, pH 7.4) was added for the reaction at room temperature for 5 minutes in order to lyse extracellular vesicles. Then, the resulting solution was magnetically collected, and the supernatant was diluted with 180 µL of Lumipulse specimen diluent (Fujirebio Inc.) to obtain "Lysis sample".

First, in order to confirm that the extracellular vesicles in the original solution sample were recovered by the immunoprecipitation method using antibodies that specifically bind to the EV surface markers (CD9, CD63 and CD81), measurement was carried out for the EV surface markers in the original solution sample and the SUP sample according to the measurement method of Example 1. As a blank, measurement was carried out for the Lumipulse specimen diluent (Fujirebio Inc.) as well. EV recovery efficiency (= (1-(SUP sample - blank)) / (original solution sample - blank) × 100 (%)) was determined based on the obtained count value.

Results are listed in Table 9. The result exhibits more than 90% of the recovery efficiency of the EV recovered by the immunoprecipitation method using each of the anti-CD9 antibody, the anti-CD63 antibody and the anti-CD81 antibody, demonstrating that the EV in the serum specimen can be recovered by the immunoprecipitation.
Also, it reveals that the recovery efficiency of the EV can be evaluated by the fully automated chemiluminescent enzyme immunoassay system.

**Table 9. CEA detection in EV in specimen**

| | Blank | Original solution sample | SUP sample | Recovery efficiency |
|---|---|---|---|---|
| IP antibody | Count | | | % |
| Anti-CD9 antibody | 980 | 16534 | 1761 | 95 |
| Anti-CD63 antibody | 6911 | 127599 | 7458 | 99.5 |
| Anti-CD81 antibody | 10843 | 7458 | 11651 | 90.5 |

The reactivity of CEA was measured in the same way as in Example 3 for the original solution sample, SUP sample and Lysis sample. The sample obtained by the immunoprecipitation using a mixture of the anti-CD9 antibody solid-phased particles, the anti-CD63 antibody solid-phased particles and the anti-CD81 antibody solid-phased particles, was used as the SUP sample and the Lysis sample in this measurement. Inhibition assay was carried out to confirm that the reactivity of CEA of the Lysis sample is the reaction specific to CEA. Specifically, an antibody that recognizes the same region as an epitope region of an anti-CEA antibody used in the Lumipulse Presto CEA reagent, was added to the CEA reagent. Then, the measurement was carried out for the CEA in the same way as in Example 3 (inhibition count).

Results are listed in Table 10. According to the result obtained by the measurement for the CEA, the original solution sample and the SUP sample exhibit a luminescent intensity of approximately 2000000 counts, revealing that a large amount of CEA is contained in the serum specimen. Meanwhile, the Lysis sample exhibits a luminescent intensity of 2025 counts, revealing that the amount of CEA derived from the EV is significantly smaller than that of CEA contained in the serum specimen as well as that it is possible to measure for the CEA derived from the EV by the method of the present invention. In the repeated measurement for the CEA, the Lysis sample exhibits 1950 counts as the count value, and this shows that the measurement is reproducible.

**Table 10. CEA detection in EV in specimen**

| | Original solution sample | SUP sample | Washing fraction 5 | Lysis sample | Inhibition count | Inhibition ratio |
|---|---|---|---|---|---|---|
| IP antibody | Count | | | | Count | % |
| CD9+CD63 +CD81 | 25,188,017 | 17,839,184 | 964 | 2,025 | 890 | 92.7 |
| CD9+CD63 +CD81 | 25,188,017 | 18,349,791 | 855 | 1,589 | 837 | 95.3 |
| Unimmobilized particles | 25,188,017 | 18,374,570 | 920 | 824 | 808 | 66.7 |

As a result of the inhibition assay, the Lysis sample exhibits a luminescent intensity of 890 counts. Since the inhibition ratio (= (1 - (inhibition count - blank count) / (count of the Lysis sample - blank count)) × 100 (%)) was 90% or more, it was confirmed that the reactivity of CEA in the Lysis sample was specific to CEA. In the same experiment repeatedly performed, the reactivity of Lysis is 1589 counts. The inhibition assay giving 837 counts achieves a inhibition ratio of 90% or more, and this shows that the experiment is reproducible.

The same test was performed using magnetic particles (unimmobilized particles) without the antibodies bound, revealing that the Lysis sample has a luminescent intensity of 824 counts without exhibiting the reactivity for the CEA. Accordingly, it reveals that unspecific adsorption on the magnetic particles of the CEA contained in the specimen can be inhibited by addition of a predetermined nonionic surfactant to the washing solution.

These results demonstrate that it is possible to specifically extract the extracellular vesicles from the human specimen and detect the CEA in the extracellular vesicles by adding the predetermined nonionic surfactant to the washing solution.

## Claims

1. A method of washing an extracellular vesicle, the method comprising washing the extracellular vesicle with a nonionic surfactant that is a chain compound containing a structure represented by -O-(-CH₂-CH₂-O-)ₓ-H, wherein x is 1 to 300, wherein the nonionic surfactant is a compound represented by formula (I): 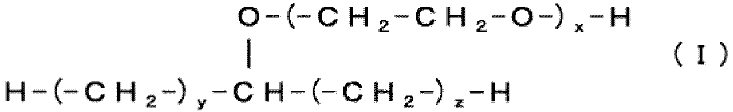
wherein x is 15 to 100;
y is an integer of 0 or more;
z is an integer of 0 or more;
y ≥ z; and
y + z is 5 to 30.

2. The method according to claim 1, wherein the alcohol ethoxylate has an HLB of 15 or more, or 16 or less carbon atoms in a carbon chain.

3. The method according to claim 1 or 2, wherein the extracellular vesicle is treated with 0.001 to 10.0 w/v% of the nonionic surfactant.

4. The method according to any one of claims 1 to 3, wherein the extracellular vesicle is exosome.

5. The method according to any one of claims 1 to 4, wherein the washing the extracellular vesicle with the nonionic surfactant is washing a complex of the extracellular vesicle and an extracellular vesicle membrane-binding substance with the nonionic surfactant.

6. The method according to claim 5, wherein the extracellular vesicle membrane-binding substance is an antibody against a tetraspanin membrane protein.

7. A method of producing a purified extracellular vesicle, the method comprising:
(1) washing an extracellular vesicle with a nonionic surfactant that is a chain compound containing a structure represented by -O-(-CH₂-CH₂-O-)ₓ-H, wherein x is 1 to 300,
wherein the nonionic surfactant is a compound represented by formula (I): 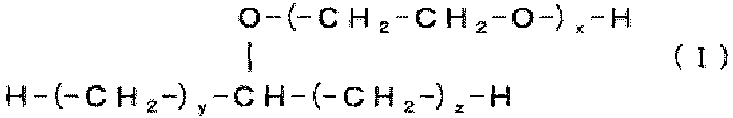
wherein x is 15 to 100;
y is an integer of 0 or more;
z is an integer of 0 or more;
y ≥ z; and
y + z is 5 to 30; and
(2) separating the washed extracellular vesicle.

8. The method according to claim 7, which is performed by the following steps:
(1') treating the extracellular vesicle with an extracellular vesicle membrane-binding substance to form a complex of the extracellular vesicle and the extracellular vesicle membrane-binding substance;
(2') washing the complex with the nonionic surfactant; and
(3') separating the complex.

9. A method of analyzing an extracellular vesicle, the method comprising analyzing the extracellular vesicle washed by the method according to any one of claims 1 to 6, or the purified extracellular vesicle produced by the method according to claim 7 or 8.

10. The method according to claim 9, which is performed by detection of an extracellular vesicle inside marker, preferably a carcinoembryonic antigen (CEA) or CA125.

11. The method according to claim 9 or 10, which is performed by the following steps:
(1) disrupting the extracellular vesicle washed by the method according to any one of claims 1 to 6, or the purified extracellular vesicle produced by the method according to claim 7 or 8; and
(2) detecting an extracellular vesicle inside marker of the disrupted extracellular vesicle.

12. A kit comprising:
(1) a nonionic surfactant that is a chain compound containing a structure represented by -O-(-CH₂-CH₂-O-)ₓ-H, wherein x is 1 to 300,
wherein the nonionic surfactant is a compound represented by formula (I): 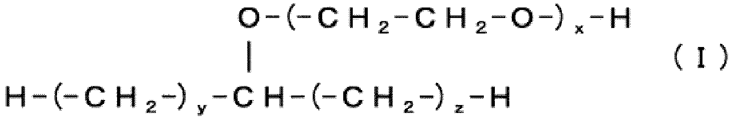
wherein x is 15 to 100;
y is an integer of 0 or more;
z is an integer of 0 or more;
y ≥ z; and
y + z is 5 to 30; and
(2) an extracellular vesicle membrane-binding substance.

## Patentansprüche

1. Verfahren zum Waschen eines extrazellulären Vesikels, wobei das Verfahren umfasst Waschen des extrazellulären Vesikels mit einem nichtionischen grenzflächenaktiven Mittel, das eine Kettenverbindung ist, die eine Struktur, dargestellt durch -O-(-CH₂-CH₂-O-)ₓ-H, enthält, wobei x 1 bis 300 ist,
wobei das nichtionische grenzflächenaktive Mittel eine Verbindung, dargestellt durch Formel (I), ist: 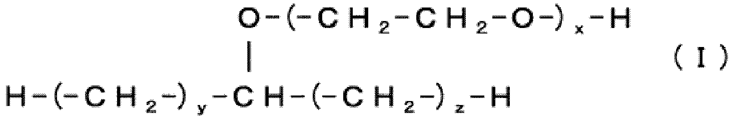
wobei x 15 bis 100 ist;
y eine ganze Zahl von 0 oder mehr ist;
z eine ganze Zahl von 0 oder mehr ist;
y ≥ z; und
y + z 5 bis 30 ist.

2. Verfahren nach Anspruch 1, wobei das Alkoholethoxylat einen HLB von 15 oder mehr oder 16 oder weniger Kohlenstoffatome in einer Kohlenstoffkette aufweist.

3. Verfahren nach Anspruch 1 oder 2, wobei das extrazelluläre Vesikel mit 0,001 bis 10,0 w/v% des nichtionischen grenzflächenaktiven Mittels behandelt wird.

4. Verfahren nach einem der Ansprüche 1 bis 3, wobei das extrazelluläre Vesikel ein Exosom ist.

5. Verfahren nach einem der Ansprüche 1 bis 4, wobei das Waschen des extrazellulären Vesikels mit dem nichtionischen grenzflächenaktiven Mittel Waschen eines Komplexes aus dem extrazellulären Vesikel und einer extrazellulären Vesikelmembran-bindenden Substanz mit dem nichtionischen grenzflächenaktiven Mittel ist.

6. Verfahren nach Anspruch 5, wobei die extrazelluläre Vesikelmembran-bindende Substanz ein Antikörper gegen ein Tetraspanin-Membranprotein ist.

7. Verfahren zur Herstellung eines gereinigten extrazellulären Vesikels, wobei das Verfahren umfasst:
(1) Waschen eines extrazellulären Vesikels mit einem nichtionischen grenzflächenaktiven Mittel, das eine Kettenverbindung ist, die eine Struktur, dargestellt durch -O-(-CH₂-CH₂-O-)ₓ-H, enthält, wobei x 1 bis 300 ist,
wobei das nichtionische grenzflächenaktive Mittel eine Verbindung, dargestellt durch Formel (I), ist: 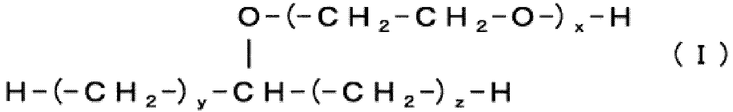
wobei x 15 bis 100 ist;
y eine ganze Zahl von 0 oder mehr ist;
z eine ganze Zahl von 0 oder mehr ist;
y ≥ z; und
y + z 5 bis 30 ist; und
(2) Separieren des gewaschenen extrazellulären Vesikels.

8. Verfahren nach Anspruch 7, das durch die folgenden Schritte durchgeführt wird:
(1') Behandeln des extrazellulären Vesikels mit einer extrazellulären Vesikelmembran-bindenden Substanz, um einen Komplex aus dem extrazellulären Vesikel und der extrazellulären Vesikelmembran-bindenden Substanz zu bilden;
(2') Waschen des Komplexes mit dem nichtionischen grenzflächenaktiven Mittel; und
(3') Separieren des Komplexes.

9. Verfahren zum Analysieren eines extrazellulären Vesikels, wobei das Verfahren umfasst Analysieren des extrazellulären Vesikels, das durch das Verfahren nach einem der Ansprüche 1 bis 6 gewaschen wurde, oder des gereinigten extrazellulären Vesikels, das durch das Verfahren nach Anspruch 7 oder 8 hergestellt wurde.

10. Verfahren nach Anspruch 9, das durch Nachweis eines extrazellulären Vesikel-Innenmarkers, vorzugsweise eines karzinoembryonalen Antigens (CEA) oder CA125, durchgeführt wird.

11. Verfahren nach Anspruch 9 oder 10, das durch die folgenden Schritte durchgeführt wird:
(1) Aufbrechen des extrazellulären Vesikels, das durch das Verfahren nach einem der Ansprüche 1 bis 6 gewaschen wurde, oder des gereinigten extrazellulären Vesikels, das durch das Verfahren nach Anspruch 7 oder 8 hergestellt wurde; und
(2) Nachweisen eines extrazellulären Vesikel-Innenmarkers des aufgebrochenen extrazellulären Vesikels.

12. Kit, umfassend:
(1) ein nichtionisches grenzflächenaktives Mittel, das eine Kettenverbindung ist, die eine Struktur, dargestellt durch -O-(-CH₂-CH₂-O-)ₓ-H, enthält, wobei x 1 bis 300 ist,
wobei das nichtionische grenzflächenaktive Mittel eine Verbindung, dargestellt durch Formel (I), ist: 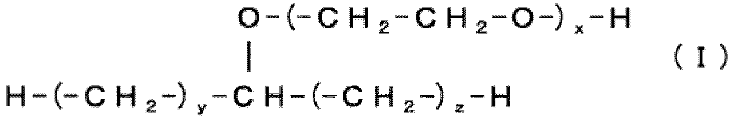
wobei x 15 bis 100 ist;
y eine ganze Zahl von 0 oder mehr ist;
z eine ganze Zahl von 0 oder mehr ist;
y ≥ z; und
y + z 5 bis 30 ist; und
(2) eine extrazelluläre Vesikelmembran-bindende Substanz.

## Revendications

1. Procédé de lavage d'une vésicule extracellulaire, le procédé comprenant laver la vésicule extracellulaire avec un tensioactif non ionique qui est un composé à chaîne contenant une structure représentée par -O-(-CH₂-CH₂-O)ₓ-H, dans laquelle x est 1 à 300,
dans lequel le tensioactif non ionique est un composé représenté par la formule (I) : 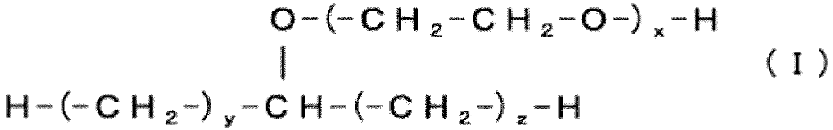
dans laquelle x est 15 à 100 ;
y est un entier de 0 ou plus ;
z est un entier de 0 ou plus ;
y ≥ z ; et
y + z est 5 à 30.

2. Procédé selon la revendication 1, dans lequel l'éthoxylate d'alcool a une HLB de 15 ou plus, ou 16 ou moins atomes de carbone dans une chaîne de carbone.

3. Procédé selon la revendication 1 ou 2, dans lequel la vésicule extracellulaire est traitée avec 0,001 à 10,0 % en p/v du tensioactif non ionique.

4. Procédé selon l'une quelconque des revendications 1 à 3, dans lequel la vésicule extracellulaire est un exosome.

5. Procédé selon l'une quelconque des revendications 1 à 4, dans lequel le lavage de la vésicule extracellulaire avec le tensioactif non ionique est un lavage d'un complexe de la vésicule extracellulaire et d'une substance de liaison à la membrane de vésicule extracellulaire avec le tensioactif non ionique.

6. Procédé selon la revendication 5, dans lequel la substance de liaison à la membrane de vésicule extracellulaire est un anticorps contre une protéine membranaire de tétraspanine.

7. Procédé de production d'une vésicule extracellulaire purifiée, le procédé comprenant :
(1) laver une vésicule extracellulaire avec un tensioactif non ionique qui est un composé à chaîne contenant une structure représentée par -O-(-CH₂-CH₂-O)ₓ-H, dans laquelle x est 1 à 300,
dans lequel le tensioactif non ionique est un composé représenté par la formule (I) : 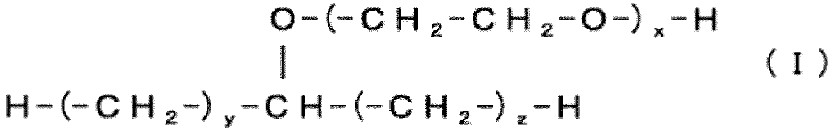
dans laquelle x est 15 à 100 ;
y est un entier de 0 ou plus ;
z est un entier de 0 ou plus ;
y ≥ z ; et
y + z est 5 à 30 ; et
(2) séparer la vésicule extracellulaire lavée.

8. Procédé selon la revendication 7, qui est réalisé par les étapes suivantes :
(1') traiter la vésicule extracellulaire avec une substance de liaison à la membrane de vésicule extracellulaire pour former un complexe de la vésicule extracellulaire et la substance de liaison à la membrane de vésicule extracellulaire ;
(2') laver le complexe avec le tensioactif non ionique ; et
(3') séparer le complexe.

9. Procédé d'analyse d'une vésicule extracellulaire, le procédé comprenant analyser la vésicule extracellulaire lavée par le procédé selon l'une quelconque des revendications 1 à 6, ou la vésicule extracellulaire purifiée produite par le procédé selon la revendication 7 ou 8.

10. Procédé selon la revendication 9, qui est réalisé par détection d'un marqueur intérieur de vésicule extracellulaire, de préférence un antigène carcino-embryonnaire (ACE) ou CA125.

11. Procédé selon la revendication 9 ou 10, qui est réalisé par les étapes suivantes :
(1) perturber la vésicule extracellulaire lavée par le procédé selon l'une quelconque des revendications 1 à 6, ou la vésicule extracellulaire purifiée produite par le procédé selon la revendication 7 ou 8 ; et
(2) détecter un marqueur intérieur de vésicule extracellulaire de la vésicule extracellulaire perturbée.

12. Kit comprenant :
(1) un tensioactif non ionique qui est un composé à chaîne contenant une structure représentée par -O-(-CH₂-CH₂-O)ₓ-H, dans laquelle x est 1 à 300,
dans lequel le tensioactif non ionique est un composé représenté par la formule (I) : 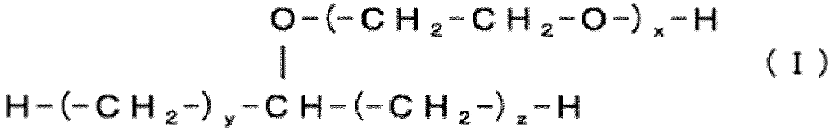
dans laquelle x est 15 à 100 ;
y est un entier de 0 ou plus ;
z est un entier de 0 ou plus ;
y ≥ z ; et
y + z est 5 à 30 ; et
(2) une substance de liaison à la membrane de vésicule extracellulaire.
